(19) Europäisches Patentamt
European Patent Office
Office européen des brevets

(11) EP 1 781 238 B1

(12) **EUROPEAN PATENT SPECIFICATION**

(45) Date of publication and mention of the grant of the patent:
**18.03.2009 Bulletin 2009/12**

(21) Application number: **05770137.7**

(22) Date of filing: **13.07.2005**

(51) Int Cl.:
***A61K 6/087*** *(2006.01)*

(86) International application number:
**PCT/US2005/024822**

(87) International publication number:
**WO 2006/019797 (23.02.2006 Gazette 2006/08)**

(54) **DENTAL COMPOSITIONS CONTAINING OXIRANE MONOMERS**

DENTALZUSAMMENSETZUNGEN MIT OXIRAN-MONOMEREN

COMPOSITIONS POUR CHIRURGIE DENTAIRE CONTENANT DES MONOMÈRES DE TYPE OXIRANE

(84) Designated Contracting States:
**AT BE BG CH CY CZ DE DK EE ES FI FR GB GR HU IE IS IT LI LT LU LV MC NL PL PT RO SE SI SK TR**

(30) Priority: **14.07.2004 US 587977 P**

(43) Date of publication of application:
**09.05.2007 Bulletin 2007/19**

(73) Proprietors:
- **3M Innovative Properties Company**
  **St. Paul MN 55133-3427 (US)**
- **3M ESPE AG**
  **82229 Seefeld (DE)**

(72) Inventors:
- **MADER, Roger A.**
  **3M Center**
  **Saint Paul, MN 55133-3427 (US)**
- **JACOBS, Dwight W.**
  **3M Center**
  **Saint Paul, MN 55133-3427 (US)**
- **ECKERT, Adrian S.**
  **3M Center**
  **Saint Paul, MN 55133-3427 (US)**

(74) Representative: **Brem, Roland et al**
**3M ESPE AG**
**-IP Department-**
**ESPE Platz**
**82229 Seefeld (DE)**

(56) References cited:
**EP-A- 0 748 831**
**WO-A-99/03444**
**US-A- 2 965 607**
**US-A1- 2002 151 731**
**US-B1- 6 245 828**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 781 238 B1

## Description

FIELD OF THE INVENTION

Dental compositions comprising oxirane monomers.

BACKGROUND

Except for glass ionomers, all organic based dental restoratives to date are based on methacrylate/acrylate chemistries, which exhibit unacceptably high stress upon curing due to their excessively high polymerization shrinkage. Oxirane chemistries have been demonstrated to exhibit lower shrinkage and lower polymerization stress than the best methacrylate-based materials on the market.

However, to date the refractive index of certain oxirane systems do not ideally matched the refractive index (RI) of preferred fillers (quartz/radiopaque melt glasses/radiopaque sol gel fillers). As a result, the translucency (aesthetics) of the composites made with such oxirane systems is not as high as desired.

Also the Polymerization Stress and Post Gel Shrinkage (strain gage method) of certain oxirane restorative systems is only about half of that of the lowest shrinking methacrylate-based restorative materials on the market today.

Thus, there is still a need for new components that can be added to oxirane-based dental compositions, or used alone, with good aesthetics, mechanical properties, and low shrinkage. WO 99/03444 discloses dental compositions comprising epoxides.

SUMMARY OF THE INVENTION

The present invention provides epoxy-functional ether monomers (i.e., oxirane monomers) for use in dental compositions. Significantly, one or more of the monomers of the current invention when used in combination with other oxirane compounds, can provide one or more of the following: improved aesthetics, improved mechanical strength, and higher degree of conversion, without significantly detrimentally affecting shrinkage or other physical properties of the composites.

The epoxy-functional ether monomer preferably has the following Formula (I):

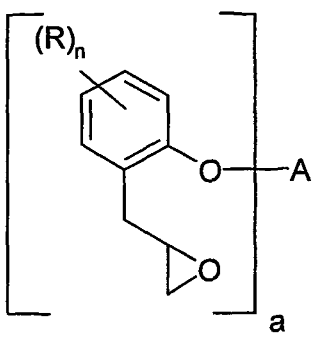

(I)

wherein: A represents a straight- or branched-chain alkylene, cycloalkylene, arylalkylene, or arylcycloalkylene, each having 1-30 carbon atoms, wherein one or more carbon and/or hydrogen atoms are optionally substituted by one or more Br, Cl, N, or O atoms, or combinations thereof; each R independently represents an alkyl, aryl, or epoxyalkyl; n is 0-4; and a is 2-4.

These monomers can be used alone as the resin system in a dental composition or in combination with other resin system reactive components typically used in oxirane-based systems, such as epoxy compounds including aromatic groups, aliphatic groups, cycloaliphatic groups, and combinations thereof.

The compositions also typically include an initiator system. The initiator system can include one or more initiators. Such initiators are preferably capable of inducing a cationic ring opening polymerization reaction.

In certain embodiments, dental compositions also include a filler system. Other optional ingredients include, for example, a colorant, a surfactant, a flavoring agent, a medicament, a stabilizer, a viscosity modifier, a diluting agent, a flow control additive, a thixotropic agent, an antimicrobial, and a polymeric thickener.

Various combinations of each of the components listed herein can be used for desired effect.

The terms "comprises" and variations thereof do not have a limiting meaning where these terms appear in the description and claims.

As used herein, "a," "an," "the," "at least one," and "one or more" are used interchangeably. Thus, for example, a dental composition that comprises "a" oxirane-containing monomer can be interpreted to mean that the dental composition includes "one or more" oxirane-containing monomers. Similarly, a composition comprising "a" filler can be interpreted to mean that the composition includes "one or more" types of fillers.

Also herein, the recitations of numerical ranges by endpoints include all numbers subsumed within that range (e.g., 1 to 5 includes 1, 1.5, 2, 2.75, 3, 3.80, 4, 5, etc.).

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

## DETAILED DESCRIPTION OF ILLUSTRATIVE EMBODIMENTS

The present invention provides epoxy-functional ether monomers for use in dental compositions. These monomers can be used alone as the resin system in a dental composition or in combination with other resin system reactive components typically used in oxirane-based systems, such as epoxy compounds including aromatic groups, aliphatic groups, cycloaliphatic groups, and combinations thereof.

### Epoxy-functional Ether Monomers and Preparation Thereof

Preferred epoxy-functional ether monomers of the present invention have Formula (I):

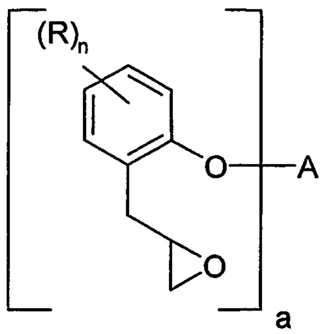

(I)

wherein: A represents a straight- or branched-chain alkylene, cycloalkylene, arylalkylene, or arylcycloalkylene, each having 1-30 carbon atoms, wherein one or more carbon and/or hydrogen atoms are optionally substituted by one or more Br, Cl, N, or O atoms, or combinations thereof; each R independently represents an alkyl, aryl, or epoxyalkyl (if n = 0 and R is not present, the open carbons of the aromatic ring are substituted by H atoms); n is 0-4; and a is 2-4. Herein, it will be clear to one of skill in the art that the R groups do not include halogens.

Preferably, a is 3, and more preferably, a is 2. Preferably, n is 0 (and all the open carbon atoms are substituted by H atoms). Preferably, A does not include any halogens. More preferably, A represents an alkylene.

According to Formula (I) the following compounds are preferred examples of epoxy functional ether derivatives:

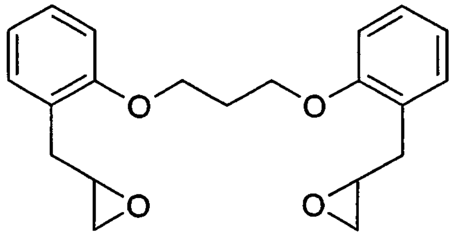

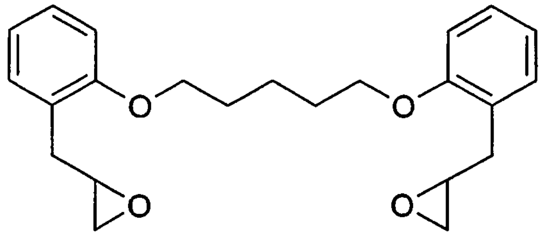

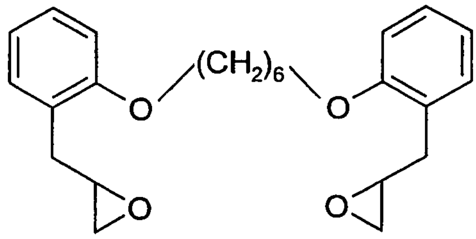

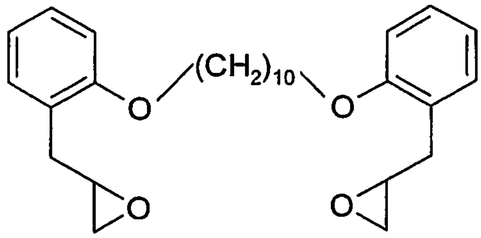

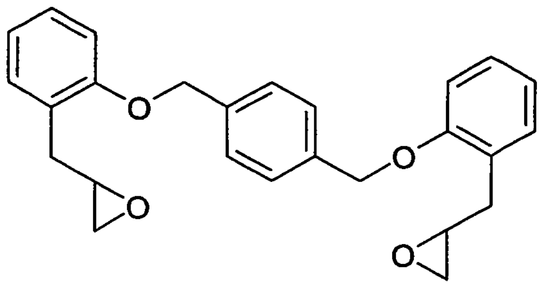

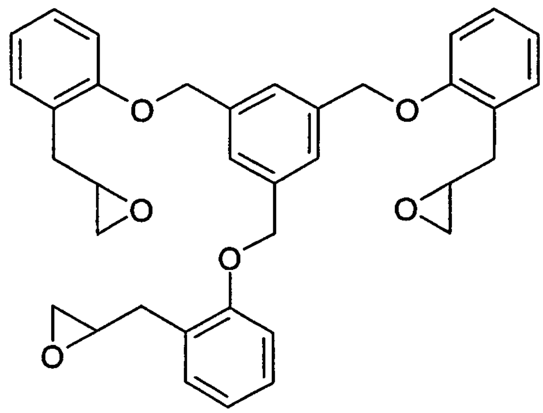

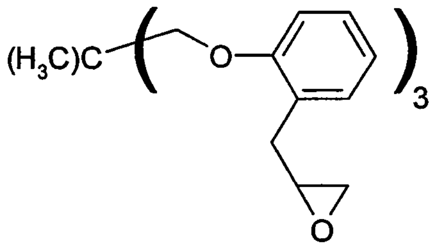

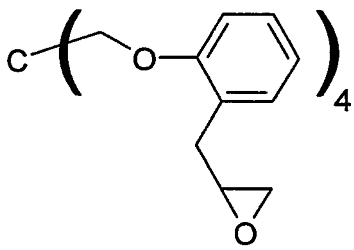

Preparation of Compounds

The epoxy-functional ether monomers of the present invention can be prepared by the following scheme utilizing generally commercially available (e.g. from Sigma-Aldrich, St. Louis, MO) 2-allylphenol and derivatives thereof as starting materials and MCPBA (m-chloroperbenzoic acid) to convert the olefin moieties into epoxides. The scheme illustrates the general preparation of compounds such as represented by Formula (II), (i.e., Formula (I), wherein a = 2).

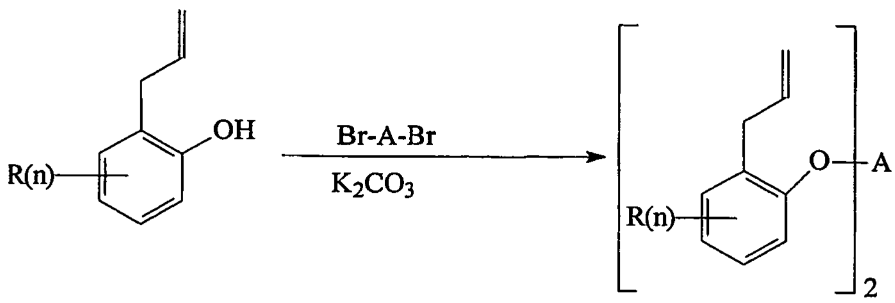


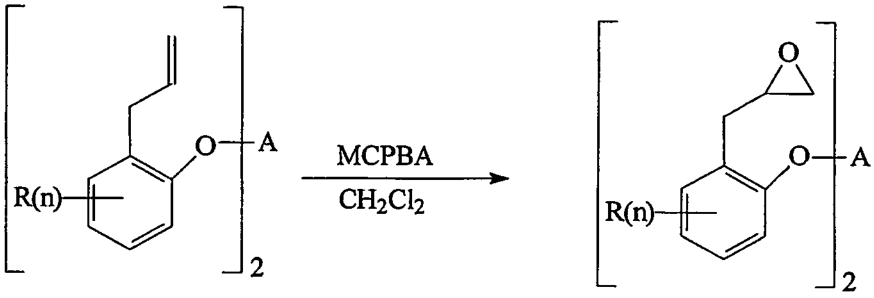


(II)

Examples 1-4 (Compounds A-D) in the Examples Section detail the preparation of Formula (II), wherein n = 0, and A = $(CH_2)_x$, where x = 3, 5, 6, or 10, respectively.

The epoxy-functional ether monomers can be formulated into dental composites that exhibit a total volumetric polymerization shrinkage of no greater than 2.0% (typically, a shrinkage of 1.0% to 2.0%), wherein the percentage is based on the volume of the composition prior to hardening, while maintaining excellent physical properties.

Monomers of the present invention can be used in an amount of up to 100% in dental compositions, depending on the use of the composition. Preferably, the total amount of the epoxy-functional ether monomers in a dental composition is at least 1 percentage by weight (wt-%), more preferably, at least 3 wt-%, and most preferably, at least 5 wt-%, based on the total weight of the composition. Preferably, the total amount of the epoxy-functional ether monomers is no greater than 80 wt-%, more preferably, no greater than 60 wt-%, and most preferably, no greater than 40 wt-%, based on the total weight of the composition.

Oxirane Resin Systems

The epoxy-functional ether monomers of the present invention can be used alone as the resin system in a dental composition or in combination with other resin system reactive components typically used in oxirane-based systems, such as epoxy compounds including aromatic groups, aliphatic groups, cycloaliphatic groups, and combinations thereof.

Suitable resin system reactive components (i.e., photopolymerizable materials and compositions) may include epoxy resins (which contain cationically active epoxy groups), vinyl ether resins (which contain cationically active vinyl ether groups), ethylenically unsaturated compounds (which contain free radically active unsaturated groups), and combinations thereof. Examples of useful ethylenically unsaturated compounds include acrylic acid esters, methacrylic acid esters, hydroxy-functional acrylic acid esters, hydroxy-functional methacrylic acid esters, and combinations thereof. Also suitable are polymerizable materials that contain both a cationically active functional group and a free radically active functional group in a single compound. Examples include epoxy-functional acrylates, epoxy-functional methacrylates, and combinations thereof.

Resin system reactive components may include compounds having free radically active functional groups that may include monomers, oligomers, and polymers having one or more ethylenically unsaturated group. Suitable compounds contain at least one ethylenically unsaturated bond and are capable of undergoing addition polymerization. Such free radically polymerizable compounds include (meth)acrylates (i.e., acrylates and methacrylates) and (meth)acrylamides (i.e., acrylamides and methacrylamides), for example. Specific examples include mono-, di- or poly-acrylates and methacrylates such as described in U.S. Pat. No. 6,187,836 (Oxman et al.)

Resin system reactive components may include compounds having cationically active functional groups such

as cationically polymerizable epoxy resins. Such polymerizable materials include organic compounds having an oxirane ring that is polymerizable by ring opening. These materials include monomeric epoxy compounds and epoxides of the polymeric type and can be aliphatic, cycloaliphatic, aromatic or heterocyclic. These compounds generally have, on the average, at least 1 polymerizable epoxy group per molecule, in some embodiments at least about 1.5, and in other embodiments at least about 2 polymerizable epoxy groups per molecule. The polymeric epoxides include linear polymers having terminal epoxy groups (e.g., a diglycidyl ether of a polyoxyalkylene glycol), polymers having skeletal oxirane units (e.g., polybutadiene polyepoxide), and polymers having pendent epoxy groups (e.g., a glycidyl methacrylate polymer or copolymer). The epoxides may be pure compounds or may be mixtures of compounds containing one, two, or more epoxy groups per molecule. The "average" number of epoxy groups per molecule is determined by dividing the total number of epoxy groups in the epoxy-containing material by the total number of epoxy-containing molecules present.

These epoxy-containing materials may vary from low molecular weight monomeric materials to high molecular weight polymers and may vary greatly in the nature of their backbone and substituent groups. Illustrative of permissible substituent groups include halogens, ester groups, ethers, sulfonate groups, siloxane groups, carbosilane groups, nitro groups, phosphate groups, and the like. The molecular weight of the epoxy-containing materials may vary from about 58 to about 100,000 or more.

Suitable epoxy-containing materials useful as the resin system reactive components in the present invention are listed in U.S. Pat. Nos. 6,187,836 (Oxman et al.) and 6,084,004 (Weinmann et al.).

Other suitable epoxy resins useful as the resin system reactive components include those which contain cyclohexene oxide groups such as epoxycyclohexanecarboxylates, typified by 3,4-epoxycyclohexylmethyl-3,4-epoxycyclohexanecarboxylate, 3,4-epoxy-2-methylcyclohexylmethyl-3,4-epoxy-2-methylcyclohexane carboxylate, and bis(3,4-epoxy-6-methylcyclohexylmethyl) adipate. For a more detailed list of useful epoxides of this nature, reference is made to U.S. Patent Nos. 6,245,828 (Weinmann et al.) and 5,037,861 (Crivello et al.); and U.S. Patent Publication No. 2003/035899 (Klettke et al.). Other epoxy resins that may be useful in the compositions of this invention include glycidyl ether monomers. Examples are glycidyl ethers of polyhydric phenols obtained by reacting a polyhydric phenol with an excess of chlorohydrin such as epichlorohydrin (e.g., the diglycidyl ether of 2,2-bis-(2,3-epoxypropoxyphenol)propane). Further examples of epoxides of this type are described in U.S. Patent No. 3,018,262 (Schroeder), and in "Handbook of Epoxy Resins" by Lee and Neville, McGraw-Hill Book Co., New York (1967).

Particularly suitable epoxides useful as the resin system reactive components are those that contain silicon, useful examples of which are described in International Patent Publication No. WO 01/51540 (Klettke et al.).

Additional suitable epoxides useful as the resin system reactive components, including numerous commercially available epoxides, are provided in U.S. Publication No. 2005-0113477 A1, published May 26, 2005.

Blends of various epoxy-containing materials are also contemplated. Examples of such blends include two or more weight average molecular weight distributions of epoxy-containing compounds, such as low molecular weight (below 200), intermediate molecular weight (about 200 to 10,000) and higher molecular weight (above about 10,000). Alternatively or additionally, the epoxy resin may contain a blend of epoxy-containing materials having different chemical natures, such as aliphatic and aromatic, or functionalities, such as polar and non-polar.

Other types of useful resin system reactive components having cationically active functional groups include vinyl ethers, oxetanes, spiro-orthocarbonates, spiro-orthoesters, and the like.

If desired, both cationically active and free radically active functional groups may be contained in a single molecule. Such molecules may be obtained, for example, by reacting a di- or poly-epoxide with one or more equivalents of an ethylenically unsaturated carboxylic acid. An example of such a material is the reaction product of UVR-6105 (available from Union Carbide) with one equivalent of methacrylic acid. Commercially available materials having epoxy and free-radically active functionalities include the CYCLOMER series, such as CYCLOMER M-100, M-101, or A-200 available from Daicel Chemical, Japan, and EBECRYL-3605 available from Radcure Specialties, UCB Chemicals, Atlanta, GA

The cationically curable resin system reactive components may further include a hydroxyl-containing organic material. Suitable hydroxyl-containing materials may be any organic material having hydroxyl functionality of at least 1, and preferably at least 2. Preferably, the hydroxyl-containing material contains two or more primary or secondary aliphatic hydroxyl groups (i.e., the hydroxyl group is bonded directly to a non-aromatic carbon atom). The hydroxyl groups can be terminally situated, or they can be pendent from a polymer or copolymer. The molecular weight of the hydroxyl-containing organic material can vary from very low (e.g., 32) to very high (e.g., one million or more). Suitable hydroxyl-containing materials can have low molecular weights, i.e., from about 32 to about 200, intermediate molecular weights, i.e., from about 200 to about 10,000, or high molecular weights, i.e., above about 10,000. As used herein, all molecular weights are weight average molecular weights.

The hydroxyl-containing materials may be non-aromatic in nature or may contain aromatic functionality. The hydroxyl-containing material may optionally contain heteroatoms in the backbone of the molecule, such as nitrogen, oxygen, sulfur, and the like. The hydroxyl-containing material may, for example, be selected from naturally occurring or synthetically prepared cellulosic materials. The hydroxyl-containing material should be substantially free of groups which

may be thermally or photolytically unstable; that is, the material should not decompose or liberate volatile components at temperatures below about 100°C or in the presence of actinic light which may be encountered during the desired photopolymerization conditions for the polymerizable compositions.

Suitable hydroxyl-containing materials useful in the present invention are listed in U.S. Pat. No. 6,187,836 (Oxman et al.).

The amount of hydroxyl-containing organic material used in the polymerizable compositions may vary over broad ranges, depending upon factors such as the compatibility of the hydroxyl-containing material with the cationically and/or free radically polymerizable component, the equivalent weight and functionality of the hydroxyl-containing material, the physical properties desired in the final composition, the desired speed of polymerization, and the like.

Blends of various hydroxyl-containing materials may also be used. Examples of such blends include two or more molecular weight distributions of hydroxyl-containing compounds, such as low molecular weight (below about 200), intermediate molecular weight (about 200 to about 10,000) and higher molecular weight (above about 10,000). Alternatively, or additionally, the hydroxyl-containing material may contain a blend of hydroxyl-containing materials having different chemical natures, such as aliphatic and aromatic, or functionalities, such as polar and non-polar. As an additional example, one may use mixtures of two or more poly-functional hydroxy materials or one or more monofunctional hydroxy materials with poly-functional hydroxy materials.

The resin system reactive component(s) may also contain hydroxyl groups and free radically active functional groups in a single molecule. Examples of such materials include hydroxyalkylacrylates and hydroxyalkylmethacrylates such as hydroxyethylacrylate, hydroxyethylmethacrylate; glycerol mono- or di-(meth)acrylate; trimethylolpropane mono- or di-(meth)acrylate, pentaerythritol mono-, di-, and tri-(meth)acrylate, sorbitol mono-, di-, tri-, tetra-, or penta-(meth) acrylate; and 2,2-bis[4-(2-hydroxy-3 methacryloxypropoxy)phenyl]propane.

The resin system reactive component(s) may also contain hydroxyl groups and cationically active functional groups in a single molecule. An example is a single molecule that includes both hydroxyl groups and epoxy groups.

Initiator System

Compositions of the present invention include an initiator system, i.e., one initiator or a mixture of two or more initiators, which are suitable for hardening (e.g., polymerizing and/or crosslinking) an oxirane resin system.

Such initiators can be light curing or chemical curing or redox curing. Examples of such initiators are Lewis or Broensted acids, or compounds which liberate such acids, which initiate the polymerization, for example $BF_3$ or ether adducts thereof ($BF_3 \cdot THF$, $BF_3 \cdot Et_2O$, etc.), $AlCl_3$, $FeCl_3$, $HPF_6$, $HAsF_6$, $HSbF_6$, or $HBF_4$, or substances which initiate the polymerization after irradiation by UV or visible light or by means of heat and/or pressure, such as, for example, ($\eta^6$-cumene)($\eta^5$-cyclopentadienyl)iron hexafluorophosphate, ($\eta^6$-cumene) ($\eta^5$-cyclopentadienyl)iron tetrafluoroborate, ($\eta^6$-cumene) ($\eta^5$-cyclopentadienyl)iron hexafluoroantimonate, substituted diaryliodonium salts and triarylsulphonium salts. Accelerators which can be employed are peroxy compounds of the perester, diacyl peroxide, peroxydicarbonate and hydroperoxide type. Hydroperoxides are preferably used. Cumene hydroperoxide in an approximately 70% to 90% solution in cumene is employed as the particularly preferred accelerator. The ratio of photoinitiator to cumene hydroperoxide can be varied within wide limits from 1:0.001 to 1:10, but the ratio preferably used is 1:0.1 to 1:6, and most preferably 1:0.5 to 1:4. The use of complexing agents, such as, oxalic acid, 8-hydroxyquinoline, ethylenediaminetetraacetic acid and aromatic polyhydroxy compounds, is also possible.

Suitable photoinitiators for polymerizing cationically photopolymerizable compositions include binary and tertiary systems. Typical tertiary photoinitiators include an iodonium salt, a photosensitizer, and an electron donor compound as described in EP 0 897 710 (Weinmann et al.); in U.S. Pat. Nos. 5,856,373 (Kaisaki et al.), 6,084,004 (Weinmann et al.), 6,187,833 (Oxman et al.), and 6,187,836 (Oxman et al.); in U.S. Publication Number 2003/0166737 (Dede et al.); and in U.S. Publication No. 2005-0113477 A1, published May 26, 2005.

Suitable iodonium salts include tolylcumyliodonium tetrakis(pentafluorophenyl)borate, tolylcumyliodonium tetrakis(3,5-bis(trifluoromethyl)-phenyl)borate, and the diaryl iodonium salts, e.g., diphenyliodonium chloride, diphenyliodonium hexafluorophosphate, diphenyliodonium hexafluoroantimonate, and diphenyliodonium tetrafluoroboarate. Suitable photosensitizers are monoketones and diketones that absorb some light within a range of about 450 nanometers (nm) to about 520 nm (preferably, about 450 nm to about 500 nm). More suitable compounds are alpha diketones that have some light absorption within a range of about 450 nm to about 520 nm (even more preferably, about 450 nm to about 500 nm). Preferred compounds are camphorquinone, benzil, furil, 3,3,6,6-tetramethylcyclohexanedione, phenanthraquinone and other cyclic alpha diketones. Most preferred is camphorquinone. Suitable electron donor compounds include substituted amines, e.g., ethyl 4-(dimethylamino)benzoate and 2-butoxyethyl 4-(dimethylamino)benzoate; and polycondensed aromatic compounds (e.g. anthracene).

The initiator system is present in an amount sufficient to provide the desired rate of hardening (e.g., polymerizing and/or crosslinking). For a photoinitiator, this amount will be dependent in part on the light source, the thickness of the layer to be exposed to radiant energy, and the extinction coefficient of the photoinitiator. Preferably, the initiator system

is present in a total amount of at least 0.01 wt-%, more preferably, at least 0.03 wt-%, and most preferably, at least 0.05 wt-%, based on the weight of the composition. Preferably, the initiator system is present in a total amount of no more than 10 wt-%, more preferably, no more than 5 wt-%, and most preferably, no more than 2.5 wt-%, based on the weight of the composition.

Filler System

Compositions of the present invention can optionally include a filler system (i.e., one or more fillers). Fillers for use in the filler system may be selected from a wide variety of conventional fillers for incorporation into resin systems. Preferably, the filler system includes one or more conventional materials suitable for incorporation in compositions used for medical applications, for example, fillers currently used in dental restorative compositions. Thus, the filler systems used in the compositions of the present invention are incorporated into the resin systems.

Fillers may be either particulate or fibrous in nature. Particulate fillers may generally be defined as having a length to width ratio, or aspect ratio, of 20:1 or less, and more commonly 10:1 or less. Fibers can be defined as having aspect ratios greater than 20:1, or more commonly greater than 100:1. The shape of the particles can vary, ranging from spherical to ellipsoidal, or more planar such as flakes or discs. The macroscopic properties can be highly dependent on the shape of the filler particles, in particular the uniformity of the shape.

Preferred particulate filler is finely divided and has an average particle size (preferably, diameter) of less than 10 micrometers (i. e., microns).

Preferred micron-size particulate filler has an average particle size of at least 0.2 micron up to 1 micrometer. Nanoscopic particles have an average primary particle size of less than 200 nm (0.2 micron). The filler can have a unimodal or polymodal (e.g., bimodal) particle size distribution.

Micron-size particles are very effective for improving post-cure wear properties. In contrast, nanoscopic fillers are commonly used as viscosity and thixotropy modifiers. Due to their small size, high surface area, and associated hydrogen bonding, these materials are known to assemble into aggregated networks. Materials of this type ("nanoscopic" materials) have average primary particle sizes (i.e., the largest dimension, e.g., diameter, of unaggregated material) of no greater than 1000 nanometers (nm). Preferably, the nanoscopic particulate material has an average primary particle size of at least 2 nanometers (nm), and preferably at least 7 nm. Preferably, the nanoscopic particulate material has an average primary particle size of no greater than 50 nm, and more preferably no greater than 20 nm in size. The average surface area of such a filler is preferably at least 20 square meters per gram ($m^2/g$), more preferably, at least 50 $m^2/g$, and most preferably, at least 100 $m^2/g$.

The filler system can include an inorganic material. It can also include a crosslinked organic material that is insoluble in the polymerizable resin, and is optionally filled with inorganic filler. The filler system is preferably generally non-toxic and suitable for use in the mouth.

Suitable fillers can be radiopaque, radiolucent, or nonradiopaque. Fillers as used in dental applications are typically ceramic in nature. Examples of suitable inorganic fillers are naturally occurring or synthetic materials such as quartz, nitrides (e.g., silicon nitride), glasses derived from, for example Ce, Sb, Sn, Zr, Sr, Ba, or Al, colloidal silica, feldspar, borosilicate glass, kaolin, talc, titania, and zinc glass, zirconia-silica fillers; and low Mohs hardness fillers such as those described in U.S. Pat. No. 4,695,251 (Randklev). Examples of suitable organic filler particles include filled or unfilled pulverized polycarbonates, polyepoxides, and the like. Preferred filler particles are quartz, submicron silica, and non-vitreous microparticles of the type described in U.S. Pat. No. 4,503,169 (Randklev). Mixtures of these fillers can also be used, as well as combination fillers made from organic and inorganic materials.

An example of a suitable filler is IOTA-6 filler (Unimin Corp., Spruce Pine, NC) (milled quartz) with yttrium trifluoride for radio-opacity. A description of radiopacifying fillers, combinations of radiopacifying fillers, and combinations of radiopacifying fillers with non-radiopacifying fillers useful in cationically polymerizable compositions is also provided in U.S. Pat. No. 6,465,641 (Bretscher et al.).

Optionally, the surface of the filler particles may be treated with a surface treatment, such as a silane-coupling agent, in order to enhance the bond between the filler system and the resin system. The coupling agent may be functionalized with reactive curing groups, such as epoxies, (meth)acrylates, and the like.

Other suitable fillers are disclosed in U.S. Pat. Nos. 6,387,981 (Zhang et al.) and 6,572,693 (Wu et al.) as well as International Publication Nos. WO 01/30305 (Zhang et al.), WO 01/30306 (Windisch et al.), WO 01/30307 (Zhang et al.), and WO 03/063804 (Wu et al.). Filler components described in these references include nanosized silica particles, nanosized metal oxide particles, and combinations thereof. Nanofillers are also described in U.S. Patent Applications entitled, "Dental Compositions Containing Nanozirconia Fillers," U.S. Serial No. 10/847,782; "Dental Compositions Containing Nanofillers and Related Methods," U.S. Serial No. 10/847,781; and "Use of Nanoparticles to Adjust Refractive Index of Dental Compositions," U.S. Serial No. 10/847,803 all three of which were filed on May 17, 2004.

Preferably, the total amount of filler system is greater than 50 wt-%, more preferably, greater than 60 wt-%, and most preferably, greater than 70 wt-%, based on the total weight of the composition. Preferably, the total amount

of filler system is no more than 95 wt-%, and more preferably, no more than 80 wt-%, based on the total weight of the composition.

Optional Additives

The composition may additionally include optional agents such as colorants (e.g., pigments or dyes conventionally used for shade adjustment), surfactants, flavoring agents, medicaments, stabilizers (such as BHT), viscosity modifiers, diluting agents, flow control additives, thixotropic agents, antimicrobials, polymeric thickeners, and the like. Various combinations of these optional additives can be used if desired. Such agents may optionally include reactive functionality so that they will be copolymerized with the resin.

Preferably, the total amount of optional component is no more than 5.0 wt-%, more preferably, no more than about 2.5 wt-%, and most preferably, no more than 1.5 wt-%, based on the total weight of the composition.

Method of Use

The above described oxirane-containing monomers can be used as components in dental compositions that are hardenable. Dental compositions of the present invention can be used, for example, as dental restoratives or prosthetic devices or adhesives for orthodontic appliances (e.g., brackets and bands). Examples of restoratives include dental composites, filling materials, sealants, adhesives, and root canal filling materials. Examples of prosthetic devices include crowns (particularly, preformed chemical crowns), bridges, veneers, inlays, onlays, posts, pins, and the like.

EXAMPLES

Objects and advantages of this invention are further illustrated by the following examples, but the particular materials and amounts thereof recited in these examples, as well as other conditions and details, should not be construed to unduly limit this invention. Unless otherwise indicated, all parts and percentages are on a weight basis, all water is deionized water, and all molecular weights are weight average molecular weight.

Test Methods

Compressive Strength (CS) Test Method

Compressive strength of a test sample was measured according to modified American National Standard Institute/American Standard Association (ANSI/ASA) specification No. 27 (1993). A sample was packed into a 3.2-millimiters (mm) (inside diameter) Plexiglas tube, and the tube was capped with silicone rubber plugs and compressed axially at approximately 0.28 mega Pascal (Mpa) for 5 minutes. The sample was then light cured for 90 seconds by exposure to two oppositely disposed VISILUX Model 2500 blue light guns (3M Co., St. Paul, MN). Cured samples were cut with a diamond saw to form 6-7 mm long cylindrical plugs for measurement of compressive strength. The plugs were stored in distilled water at 37°C for 24 hours prior to testing. Measurements were carried out on an Instron tester (Instron 4505, Instron Corp., Canton, MA) with a 10 kilonewton (kN) load cell at a crosshead speed of 1 mm/minute. Five cylinders of cured samples were prepared and measured with the results reported in MPa as the average of the five measurements.

Post-Cure Flexural Strength (FS) and Flexural Modulus (FM) Test Methods

Flexural Strength and Flexural Modulus were measured according to the following test procedure. A paste sample was pressed for 5 minutes at 10 thousand pounds per square inch (kpsi) (69 MPa) at room temperature in a Delrin mold to form a 2-mm x 2-mm x 25-mm test bar. The test bar was immediately cured with two Visilux 2500 dental curing lights (3M Company) in three overlapping locations on the top side for a total of 120 seconds and further cured with two Visilux 2500 dental curing lights in two overlapping locations on the bottom side for a total of 60 additional seconds.

The bar was then sanded lightly with 600-grit sandpaper to remove flash from the molding process. After storing in distilled water at 37°C for 24 hours, the Flexural Strength and Flexural Modulus of the bar were measured on an Instron tester (Instron 4505, Instron Corp., Canton, MA) according to ANSI/ADA (American National Standard/American Dental Association) specification No. 27 (1993) at a crosshead speed of 0.75 mm/minute. Five bars of cured paste composite were prepared and measured with results reported in megapascals (MPa) as the average of the five measurements.

Gel Time Test Method

Gel Time was defined as the shortest amount of time required for a paste sample to transform into a solid such that the sample could not be easily manually dented with the tip of a spatula. The test procedure was as follows:

A test paste sample (50-70 milligrams (mg)) was placed on a sheet of white wax paper and rolled into a sphere with a mixing stick. The sample was then exposed to consecutive 2-second doses using the timer on an Elipar Trilight dental curing light (3M Company) until the sample had gelled. The light was placed as close as possible to the sample without touching. The time when the paste first gels (hardens to the point that it could not easily be dented) was recorded as the gel time.

Post-Gel Shrinkage Test Method

A test paste sample (approximately 20 mg) was shaped into a ball by rolling the sample on poly-coated paper pad with a spatula or dental placement instrument. The spherical paste sample was placed on a non-bonded biaxial strain gauge (CEA-06-032WT-120) wired to a Model 2120 signal conditioner, Micro-Measurements Group Inc., Raleigh, NC). The paste was flattened slightly and manipulated to be certain that the paste adhered well to the strain gauge active circuit element. The tip of an XL-2500 light guide (3M Company) was positioned as close as possible to the sample without touching the paste (approximately 1-mm distance). The paste sample was cured for 60 seconds with the XL-2500 light guide and the microstrain shrinkage registered by the strain gauge after 1-hour post irradiation was recorded.

Epoxy Equivalent Weight (EEW g/mole) Test Method

A paste or resin sample was weighed to 0.0001 gram (g) accuracy into a titration beaker. For resins with 200-300 EEW, 0.2 grams of sample was used; and for fillers or highly filled pastes, 4.0 grams of sample was used. The weight of sample was adjusted to obtain 1 to 9 milliliters (ml) volume dispensed at inflection point during titration with 0.1 Normal (N) perchloric acid in glacial acetic acid. Acetonitrile (25 ml) was added with pipette to the titration beaker that was then covered with Parafilm and sonicated at room temperature for 2 minutes. The sonicator liquid level was at sample solution height. Glacial acetic acid (25 ml) and 20% tetrabutylammonium bromide in glacial acetic acid (20 ml) were sequentially added with pipette to the beaker. The resulting solution was mixed with a stirring bar for 2 minutes and then titrated with a solution of 0.1N Perchloric acid in glacial acetic acid using a Mettler DL21 Titrator and Mettler DG 111-SC Electrode. The EEW of a sample was calculated a follows:

$$\text{EEW} = \text{mg of sample} / ((\text{total ml-ml of a blank}) \text{ X Normality of } HClO_4 \text{ titrant}).$$

ACTA 3-body Wear Test Method

The ACTA 3-body wear testing for a test sample compared to FILTEK Z250 Universal Restorative (3M Company) was done exactly as described in the following reference: Influence of Shearing Action of Food on Contact Stress and Subsequent Wear of Stress-bearing Composites, P. Pallav, et al., Journal of Dental Research, Jan. 1993.

Photo Differential Scanning Calorimetry (DSC) Test Method

A test sample of resin (10 mg) or paste (25 mg) was weighed into a DSC pan and the weight recorded to ± 0.00001 g. The uncured sample was placed on a sample cell and a pre-cured sample of same nominal weight was placed in the same type of DSC pan on the reference cell of a TA Instruments 2920 Photo DSC (TA Instruments, New Castle, DE). The Photo DSC program was set to equilibrate at 37°C with a slow nitrogen purge for 5 minutes and irradiation time for 1 hour at 37°C isothermal using the same slow nitrogen purge. From the resulting heat flow (Watts per gram (watts/g)) vs time curve, the time of maximum reaction rate (peak maximum time), onset of reaction (induction time) and enthalpy of reaction (area under curve during the 1-hour irradiation) was recorded.

Abbreviations, Descriptions, and Sources of Materials

| Abbreviation | Description and Source of Material |
|---|---|
| CPQ | Camphorquinone (Sigma-Aldrich, St. Louis, MO) |
| EDMAB | Ethyl 4-(N,N-dimethylamino)benzoate (Sigma-Aldrich) |
| Rhodorsil 2074 | Iodonium borate salt photoinitiator (Rhodia, Inc., Cranbury, NJ) |
| Cyracure UVR6105 | Cycloaliphatic diepoxide (used as a solvent for the CPQ and EDMAB components) (Dow Chemical, Midland, MI) |
| Poly-THF | Polytetrahydrofuran, 250 molecular weight (MW) (Sigma-Aldrich) |
| Compound A | Example 1 1,10-Bis[2-(2,3-epoxypropyl)phenoxy)decane 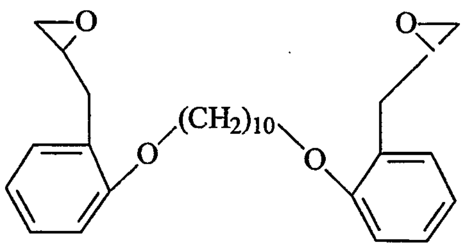 |
| Compound B | Example 2 1,3-Bis[2-(2,3-epoxypropyl)phenoxy)propane 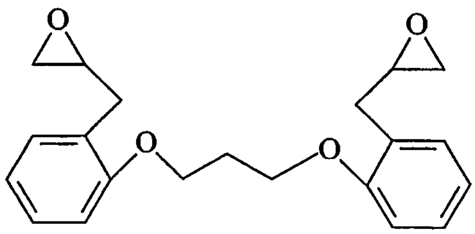 |
| Compound C | Example 3 1,5-Bis[2-(2,3-epoxypropyl)phenoxy)pentane 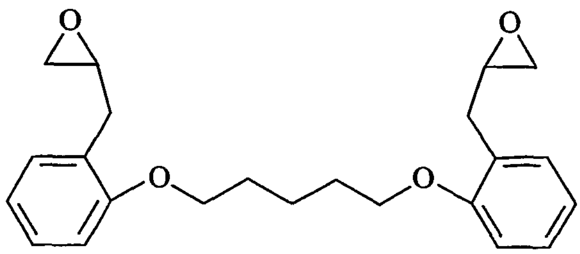 |
| Compound D | Example 4 1,6-Bis[2-(2,3-epoxypropyl)phenoxy)hexane 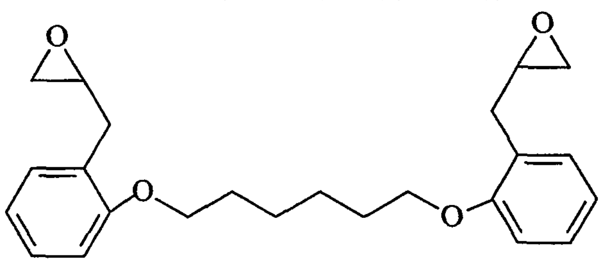 |

(continued)

| Abbreviation | Description and Source of Material |
|---|---|
| Compound E | Reference Compound [Preparatory Example 1 (Ep-Ch-E-Ester)] 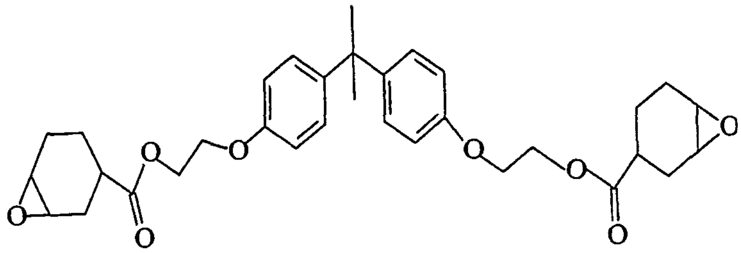 |
| Compound F | Reference Compound (Prepared according to procedures described in U.S. Patent Publication No. 2003/035899 (Kettke et al.)) Bis[2-(3,4-epoxycyclohexyl)ethyl]-methyl-phenyl-silane 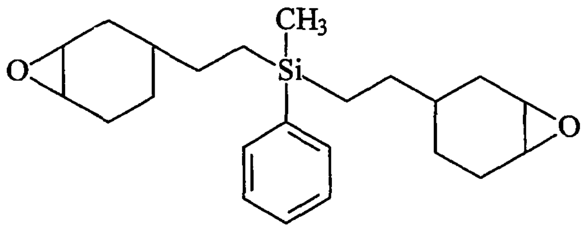 |
| Compound G | Reference Compound (Prepared according to procedures described in U.S. Patent No. 6,245,528 (Weinmann et al.)) 1,3,5,7-Tetrakis[2-(3,4-epoxycyclohexyl)ethyl]-1,3,5,7-tetramethyl-cyclotetrasiloxane 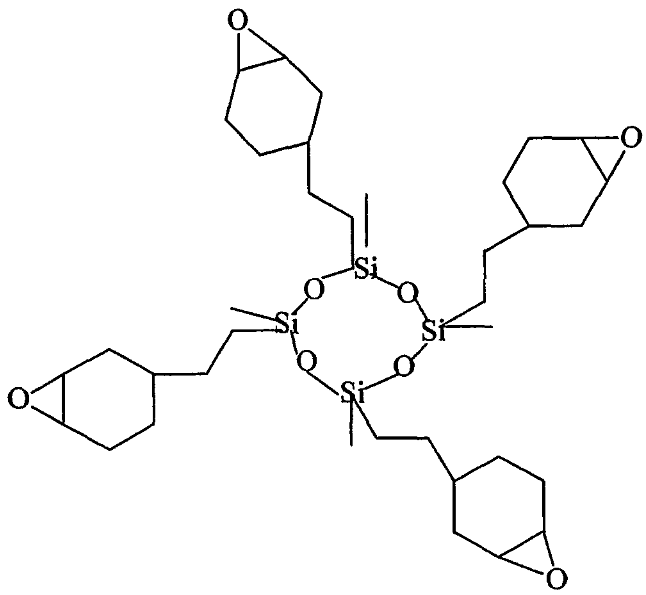 |

(continued)

| Abbreviation | Description and Source of Material |
|---|---|
| Compound H | Reference Compound (Prepared according to procedures described in U.S. Patent Publication No. 2003/035899 (Kettke et al.), U.S. Patent No. 6,245,828 (Weinmann et al.), and International Patent Publication No. WO 2002/066535 (Klettke et al.)) 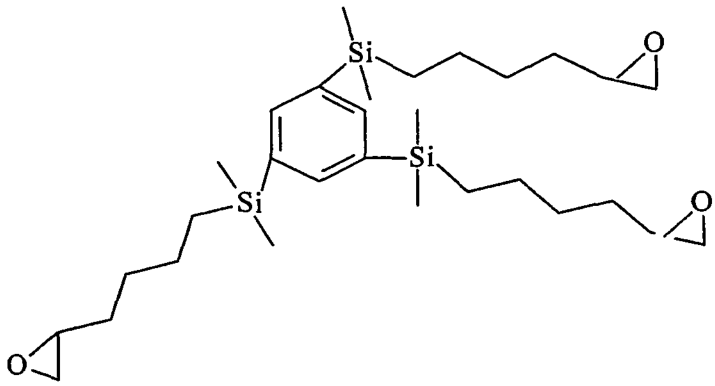 |
| Compound I | Reference Compound (Prepared according to procedures described in International Patent Publication No. WO 2002/066535 (Klettke et al.)) 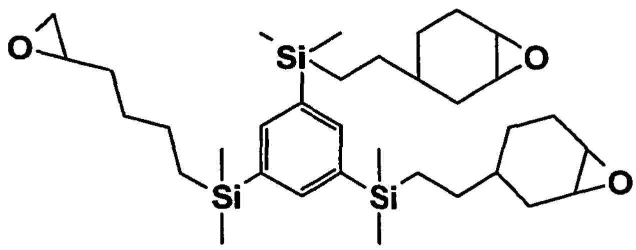 |
| Compound J | Reference Compound (ProGlyCy) (Dow Chemical, Midland, MI) 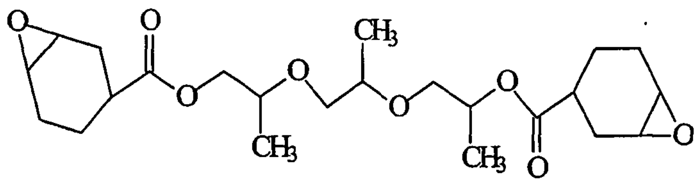 |

Example 1

Synthesis of 1,10-Bis[2-(2,3-epoxypropyl)phenoxy)decane (Compound A)

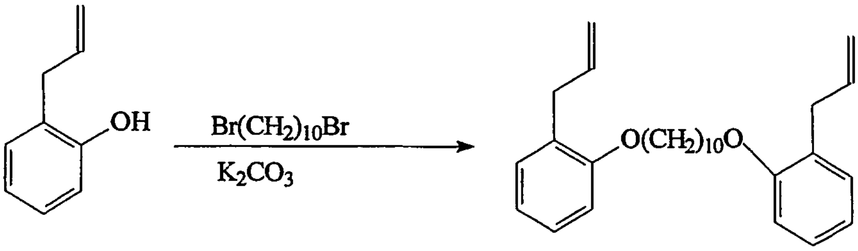


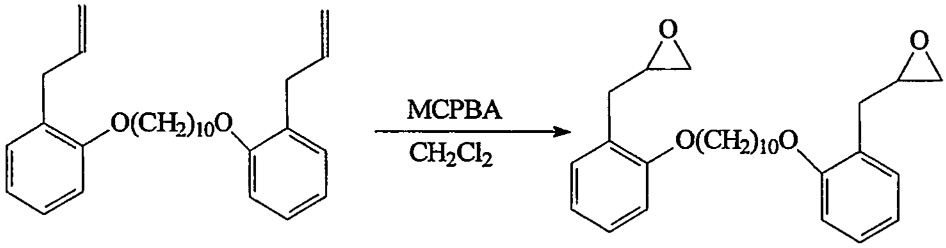


Compound A

A mixture containing 35 grams of 2-allylphenol (Sigma-Aldrich), 39 grams of 1,10-dibromodecane (Sigma-Aldrich) and 36 grams of potassium carbonate in 150 ml of methyl ethyl ketone (MEK) was heated to 80°C for 24 hours followed by the addition of 200 ml of water and 200 ml of hexane. The resulting mixture separated into an organic phase and an aqueous phase. The organic phase was washed with water and then dried over sodium sulfate. The solvent was removed and the residual liquid distilled under vacuum. The material boiling at 210-215°C (0.1 mm Hg, 13 Pascals) was collected to yield 26 grams of a liquid characterized by gas chromatography/mass spectral analysis (GC/MS) to be 1,10-bis(2-allylphenoxy)hexane.

To a solution of 1,10-bis(2-allylphenoxy)hexane (21 grams) in 210 ml of methylene chloride was added 41.6 grams of 77% m-chloroperbenzoic acid (Sigma-Aldrich). After an initial cooling caused by the dissolution of the m-chloroperbenzoic acid there was a slight exothermic reaction, and the reaction temperature was kept at 25-26°C by means of a cold-water bath. The mixture was stirred for 6 hours. Analysis of the reaction mixture by gas chromatography showed 92% of the desired product. The reaction mixture was poured into a solution containing 40 grams of sodium hydroxide in 150 ml of water and 200 ml of hexane. The resulting mixture was stirred and cooled to 10°C with an ice bath. The organic layer was removed and washed with 100 ml of 5% sodium hydrosulfite solution and three times with 100 ml of 10% potassium chloride solution. The solvent was removed and the residue was passed through a silica gel column eluted with 75% hexane 25% ethyl acetate to remove most of the color. The oil obtained was dissolved in 100 ml of 10% ethyl acetate in hexane, and was treated with 3 grams of basic aluminum oxide with stirring to remove additional color. The aluminum oxide was removed by filtration and the solvent stripped to yield 13 grams of a liquid characterized by nuclear magnetic resonance spectroscopy (NMR) to be 1,10-Bis[2-(2,3-epoxypropyl)phenoxy]decane (Compound A).

Example 2

Synthesis of 1,3-Bis[2-(2,3-epoxypropyl)phenoxy]propane (Compound B)

1,3-Bis[2-(2,3-epoxypropyl)phenoxy]propane (Compound B) was synthesized by the procedure described in Example 1, except that the 1,10-dibromodecane was replaced by 1,3-dibromopropane (Sigma-Aldrich). The overall yield for both steps was 48% and the structure of Compound B was confirmed by NMR.

Example 3

Synthesis of 1,5-Bis[2-(2,3-epoxypropyl)phenoxy]pentane (Compound C) 1,3-Bis[2-(2,3-epoxypropyl)phenoxy]pentane (Compound C) was synthesized by the procedure described in Example 1, except that the 1,10-dibromodecane was replaced by 1,5-dibromopentane (Sigma-Aldrich). The overall yield for both steps was 38% and the structure of Compound C was confirmed by NMR.

Example 4

Synthesis of 1,6-Bis[2-(2,3-epoxypropyl)phenoxy]hexane (Compound D) 1,3-Bis[2-(2,3-epoxypropyl)phenoxy] hexane (Compound D) was synthesized by the procedure described in Example 1, except that the 1,10-dibromodecane was replaced by 1,6-dibromohexane (Sigma-Aldrich). The overall yield for both steps was 25% and the structure of Compound D was confirmed by NMR.

Preparatory Compound 1 (Compound E)

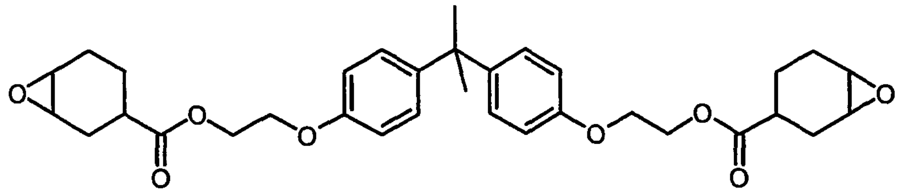

A mixture containing 50 grams of bisphenol A, 42.4 grams of ethylene carbonate, and 24 grams of triethylamine was heated to 105°C for 24 hrs. Water aspirator vacuum was applied and the mixture heated to 110°C to remove most of the triethylamine. The mixture was cooled and dissolved in 300 ml of ethyl acetate and washed with 100 ml of 5% aqueous HCl. The organic layer was then washed with 100 ml water, 100 ml 5% potassium carbonate, and 100 ml potassium chloride solution. The organic phase was washed over sodium sulfate and the solvent removed to give an oil that partially crystallized on standing. The partially crystallized oil was redissolved in 100 ml of ethyl acetate and hexane added until it became turbid. Upon standing for 2 days the material crystallized and was filtered to give 25.8 grams of 2-(4-{1-[4-(2-hydroxyethoxy)-phenyl]-1-methyl-ethyl}-phenoxy)ethanol.

A mixture containing 11 grams of the above prepared 2-(4-{1-[4-(2-hydroxyethoxy)-phenyl]-1-methyl-ethyl}-phenoxy)ethanol, 11.8 grams of 1-(7-oxa-bicyclo[4.1.0]hept-3-yl)-ethanone, (ERL414, Dow Chemical, Midland Chemical) and 0.3 grams of sodium acetate was heated to 120°C with stirring and under a nitrogen purge to aid in the removal of methanol. After 3 hours the reaction was complete as indicated by thin layer chromatography. The mixture was taken up in ethyl acetate and washed with water. The solvent was removed under vacuum and the residue extracted four times with hot hexane. The residual solvent was removed and the residue purified by column chromatography on silica gel eluted with 1:5 ethyl acetate/methylene chloride to yield 10.6 grams of product as a clear viscous liquid. The structure of Compound E was confirmed by NMR.

Examples 5 - 19 and Comparative Examples 1-4 (C1 - C4)

Mixtures of Monomers including Oxirane Monomers

A series of monomer mixtures were prepared containing oxirane monomers in combination with other oxirane monomers or in combination with silicon-containing oxirane monomers. An objective was to form monomer mixtures with refractive index (RI) values near that of quartz filler (approximately 1.54). The targeted RI value for the monomer mixtures was near 1.535 to account for the approximately 0.01 increase in RI expected upon polymerization.

To each of the prepared monomer mixtures was added a photoinitiator system to afford a resin composition comprised of the following ingredients in the weight percentages indicated: EDMAB (0.3%), CPQ (0.5%), Rhodorsil 2074 (3.0%), poly-THE (3.0%), Cyracure UVR6105 (2.2%), and oxirane monomer mixture (91.0%). To each of the resulting resin compositions was added an epoxy-functional silane-treated quartz filler (IOTA-6, 1 micrometer average particle size, Unimin Corp., Spruce Pine, NC) to afford a paste-like, light-curable (polymerizable) composition in the weight ratio of 30% resin composition and 70% filler. Mixing of the resin and filler components was accomplished with a DAC 150 FVZ Speed Mixer (Flakteck, Landrum, SC) at 3000 revolutions per minute (rpm) with short mixing cycles to avoid excessive heat generation during mixing. The resulting polymerizable paste compositions were designated Examples 5-19, and are listed in Table 1 along with Comparative Examples C1-C4 that only contained Reference Compounds. It is noted that Examples 16-19 and Comparative Example C1 only contained a single epoxy-functional monomer (Compounds A-D and Compound F, respectively) other than the Cyracure UVR6105 that was utilized as a solvent for the CPQ and EDMAB components of the photoinitiator system. Table 1 provides the relative amounts of the monomers used in the compositions and the corresponding RI values of the mixtures after addition of the photoinitiator system, but

before addition of the filler. (The corresponding Resin Compositions were designated Examples 5R-19R and Comparative Example C1-R to C4-R.)

| Table 1 Monomer Mixtures in Polymerizable Paste Compositions (Containing 30% Resin Composition and 70% Filler) | | | | |
|---|---|---|---|---|
| Ex. | Monomer Mixture (Weight % in Resin Compositions) | | | RI of Monomer Mixture (23°C) |
| 5 | Compound C (41.8) | Compound F (49.2) | - | 1.5368 |
| 6 | Compound B (71.0) | Compound J (20.0) | - | 1.5347 |
| 7 | Compound B (60.1) | Compound H (30.9) | - | 1.5333 |
| 8 | Compound B (47.3) | Compound I (43.7) | - | 1.5328 |
| 9 | Compound B (23.7) | Compound F (67.3) | - | 1.5367 |
| 10 | Compound B (67.3) | - | Compound G (23.7) | 1.5328 |
| 11 | Compound B (56.4) | Compound F (17.3) | Compound G (17.3) | 1.5359 |
| 12 | Compound B (42.2) | Compound F (24.4) | Compound I (24.4) | 1.5306 |
| 13 | Compound B (45.7) | Compound E (27.8) | Compound G (17.5) | 1.5360 |
| 14 | Compound B (37.3) | Compound E (28.1) | Compound F (12.8)<br>Compound G (12.8) | 1.5366 |
| 15 | Compound B (34.3) | Compound E (36.3) | Compound H (20.4) | 1.5342 |
| 16 | Compound A (91.0) | - | - | 1.5299 |
| 17 | Compound B (91.0) | - | - | 1.5460 |
| 18 | Compound C (91.0) | - | - | 1.5432 |
| 19 | Compound D (91.0) | - | - | 1.5410 |
| C1 | - | Compound F (91.0) | | 1.5322 |
| C2 | - | - | Compound G (91.0) | 1.4929 |
| C3 | - | Compound F (45.5) | Compound G (45.5) | 1.5138 |
| C4 | - | Compound E (60.7) | Compound F (30.3) | 1.5362 |

Evaluations

Ames Mutagenicity

Ames mutagenicity evaluations of Compounds A-D were performed by the School of Pharmacy, University of Missouri at Kansas City (UMKC). Results to date have shown Compounds A, C, and D to be negative in strains TA100 and TA97A. Compound B was negative in strains TA100, TA1535, TA98, TA102, and marginally positive in strain TA97A.

Degree of Cure (Photo Differential Scanning Calometer (DSC))

Tabulated Photo DSC results for Examples 5-19 and Comparative Examples 1-4 are shown in Table 2 (Examples as resin compositions without filler) and in Table 3 (Examples as polymerizable paste compositions with filler). Also shown in Tables 2 and 3 are:

1. the measured resin and paste epoxy equivalent weights (EEWs), in units of grams of material per mole of epoxy groups, and
2. the calculated resin and paste enthalpies in Joules per mole of epoxy groups (J/mole) in the materials.

EEW = grams per mole of epoxy groups (from titration measurements)

$$\text{Joules/mole of epoxy groups} = \text{(Joules/gram) X (grams/mole)} = \text{J/g X EEW}$$

The Joules/mole values represent the relative extent of reaction (degree of conversion) obtainable at low intensity irradiation (approximately 3 milliwatt per centimeter squared (mw/$cm^2$), a characteristic of the 3M ESPE, St. Paul, TA 2920 photo DSC).

Mechanical Properties

Examples 5-19 and Comparative Examples 1-4 (Examples as polymerizable paste compositions with filler) were evaluated for compressive strength, flexural strength and flexural modulus according to the Test Methods described herein and the test results are shown in Table 4.

Gel Time, Post-Gel Shrinkage, and ACTA 3-Body Wear

Examples 5-19 and Comparative Examples 1-4 (Examples as polymerizable paste compositions with filler) were evaluated for gel time, post-gel shrinkage, and ACTA 3-body wear according to the Test Methods described herein and the test results are shown in Table 4.

Table 2. Results of Photo DSC of Resin Compositions

| | EEW | PDSC | | | Enthalpy | |
|---|---|---|---|---|---|---|
| Resin Example Number | Average EEW (g/mole) | Average Energy (Joules/g) | Average Induction Time (min) | Average Peak Max. (min) | Average Energy (J/mol) | Propagated Error Energy (J/mol) |
| C1-R | 217 | 200 | 0.16 | 0.54 | 43363 | 414 |
| C2-R | 196 | 184 | 0.36 | 0.91 | 36025 | 2301 |
| C3-R | 216 | 203 | 0.19 | 0.58 | 43783 | 1146 |
| C4-R | 276 | 103 | 0.23 | 0.90 | 28502 | 761 |
| 5R | 216 | 265 | 0.17 | 0.89 | 57253 | 1801 |
| 6R | 201 | 293 | 0.27 | 2.80 | 58646 | 1956 |
| 7R | 191 | 353 | 0.31 | 5.34 | 67257 | 2402 |
| 8R | 203 | 314 | 0.19 | 1.21 | 63531 | 917 |
| 9R | 207 | 266 | 0.16 | 0.69 | 55235 | 454 |
| 10R | 195 | 305 | 0.24 | 1.53 | 59493 | 2264 |
| 11R | 199 | 290 | 0.19 | 1.18 | 57741 | 2757 |
| 12R | 202 | 299 | 0.26 | 1.11 | 60178 | 3735 |
| 13R | 218 | 234 | 0.24 | 1.38 | 50917 | 3599 |
| 14R | 223 | 214 | 0.22 | 1.14 | 47844 | 741 |
| 15R | 224 | 239 | 0.27 | 2.60 | 53663 | 539 |
| 16R | 235 | 319 | 0.31 | 4.88 | 74900 | 4200 |
| 17R | 191 | 320 | 0.44 | 6.65 | 61100 | 2240 |
| 18R | 205 | 356 | 0.29 | 6.9 | 72800 | 727 |
| 19R | NT* | 337 | 0.27 | 7.68 | NT | NT |
| *NT - Not Tested | | | | | | |

| Table 3. Results of DSC of Paste Compositions | | | | | | |
|---|---|---|---|---|---|---|
| | EEW | PDSC | | | Enthalpy | |
| Paste Example Number | Average EEW (g/mole) | Average Energy (Joules/g) | Average Induction Time (min) | Average Peak Max. (min) | Average Energy (J/mol) | Propagated Error Energy (J/mol) |
| C1 | 710 | 72.1 | 0.60 | 1.21 | 51212 | 2008 |
| C2 | NT | 44.9 | 0.60 | 1.63 | NT | NT |
| C3 | 748 | 52.9 | 0.52 | 1.22 | 39607 | 1047 |
| C4 | 871 | 48.4 | 0.67 | 1.66 | 42181 | 480 |
| 5 | 696 | 100.6 | 0.52 | 1.49 | 70036 | 3514 |
| 6 | 656 | 96.3 | 0.57 | 3.71 | 63128 | 1196 |
| 7 | 641 | 102.9 | 0.49 | 5.46 | 65906 | 45 |
| 8 | 656 | 84.6 | 0.52 | 1.86 | 55489 | 116 |
| 9 | 695 | 77.7 | 0.37 | 1.12 | 53969 | 1661 |
| 10 | 647 | 89.8 | 0.49 | 2.01 | 58104 | 197 |
| 11 | 667 | 86.5 | 0.47 | 1.73 | 57689 | 773 |
| 12 | 666 | 91.7 | 0.51 | 1.66 | 61020 | 1017 |
| 13 | 716 | 70.2 | 0.49 | 1.84 | 50263 | 1165 |
| 14 | 744 | 65.5 | 0.61 | 1.85 | 48709 | 121 |
| 15 | 745 | 75.6 | 0.52 | 3.36 | 56273 | 132 |
| 16 | 762 | 95.1 | 0.93 | 7.41 | 72500 | 3190 |
| 17 | 599 | 114.0 | 0.67 | 6.35 | 68300 | 1830 |
| 18 | 646 | 119.5 | 0.62 | 6.49 | 77100 | - |
| 19 | 736 | 93.37 | 1.20 | 9.99 | 68700 | - |

| Table 4. Results of Various Testing of Paste Compositions | | | | | | |
|---|---|---|---|---|---|---|
| Paste Example Number | Gel Time (sec) | CS (MPa) | Flexural Strength (MPa) | Flexural Modulus (MPa) | Post-Gel Shrinkage (*) | ACTA 3-Body Wear (Sample vs Z250) |
| C1 | 1 | 291 | 118 | 9358 | 547 | 2.11 |
| C2 | 1 | 309 | 108 | 7963 | 578 | NT |
| C3 | 2 | 306 | 122 | 8818 | 520 | 2.00 |
| C4 | 9 | 239 | 127 | 8469 | 38 | 2.99 |
| 5 | 1 | 260 | 122 | 9457 | 1158 | 2.21 |
| 6 | 7 | 276 | 140 | 10449 | 793 | 2.85 |
| 7 | 8 | 291 | 141 | 9339 | 1212 | 2.20 |
| 8 | 2 | 303 | 140 | 9328 | 937 | 2.02 |
| 9 | 1 | 260 | 147 | 10615 | 824 | 1.87 |
| 10 | 1 | 297 | 159 | 11565 | 797 | 1.85 |
| 11 | 2 | 284 | 144 | 11468 | 749 | 1.87 |

(continued)

| Table 4. Results of Various Testing of Paste Compositions | | | | | | |
|---|---|---|---|---|---|---|
| Paste Example Number | Gel Time (sec) | CS (MPa) | Flexural Strength (MPa) | Flexural Modulus (MPa) | Post-Gel Shrinkage (*) | ACTA 3-Body Wear (Sample vs Z250) |
| 12 | 2 | 293 | 153 | 10474 | 1045 | 1.79 |
| 13 | 3 | 286 | 176 | 11779 | 241 | 1.89 |
| 14 | 3 | 285 | 127 | 9945 | 163 | 2.26 |
| 15 | 7 | 304 | 132 | 7963 | 163 | 2.31 |
| 16 | 8 | NT | NT | NT | 219 | NT |
| 17 | 13 | 259 | 119 | 8759 | 896 | NT |
| 18 | 12 | NT | NT | NT | 188 | NT |
| 19 | 13 | NT | NT | NT | 61 | NT |
| (*) - Average microstrain at 3600 seconds (sec) | | | | | | |

It should be understood that this invention is not intended to be unduly limited by the illustrative embodiments and examples set forth herein and that such examples and embodiments are presented by way of example only with the scope of the invention intended to be limited only by the claims set forth herein as follows.

## Claims

**1.** A dental composition comprising:

an epoxy-functional ether monomer having Formula (I):

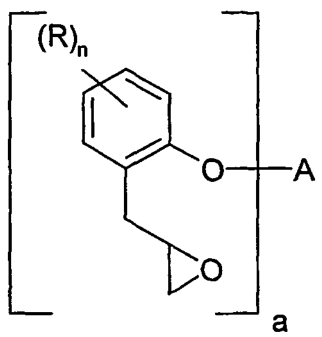

(I)

wherein:

A represents a straight- or branched-chain alkylene, cycloalkylene, arylalkylene, or arylcycloalkylene, each having 1-30 carbon atoms, wherein one or more carbon and/or hydrogen atoms are optionally substituted by one or more Br, Cl, N, or O atoms, or combinations thereof;
each R independently represents alkyl, aryl, or epoxyalkyl;
n is 0-4; and
a is 2-4; and

an initiator system.

2. The dental composition of claim 1 wherein a is 3.

3. The dental composition of claim 1 wherein a is 2.

4. The dental composition of any one of claims 1 through 3 wherein A represents an alkylene.

5. The dental composition of claim 4 wherein A represents an alkylene having 3-10 carbon atoms.

6. The dental composition of any one of claims 1 through 5 wherein n is 0.

7. The dental composition of any one of claims 1 through 6 further comprising secondary resin system reactants selected from the group consisting of epoxy compounds including aromatic groups, aliphatic groups, cycloaliphatic groups, and combinations thereof.

8. The dental composition of claim 7 wherein the secondary resin system further comprises silicon-containing reactants.

9. The dental composition of any one of claims 1 through 8 further comprising a filler system.

10. The dental composition of any one of claims 1 through 9 further comprising an additive selected from the group consisting of a colorant, a surfactant, a flavoring agent, a medicament, a stabilizer, a viscosity modifier, a diluting agent, a flow control additive, a thixotropic agent, an antimicrobial, a polymeric thickener, and combinations thereof.

**Patentansprüche**

1. Dentalmasse, umfassend:

   ein epoxidfunktionelles Ethermonomer der Formel (I) :

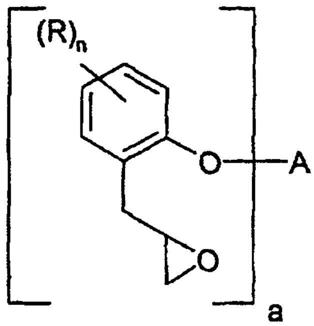

(I)

worin:

A für ein gerad- oder verzweigtkettiges Alkylen, Cycloalkylen, Arylalkylen oder Arylcycloalkylen mit jeweils 1-30 Kohlenstoffatomen steht, wobei ein oder mehrere Kohlenstoff- und/oder Wasserstoffatome gegebenenfalls durch ein oder mehrere Br-, Cl-, N- oder O-Atome oder Kombinationen davon ersetzt sind;
jedes R unabhängig für Alkyl, Aryl oder Epoxyalkyl steht;
n für 0-4 steht und
a für 2-4 steht; und
ein Initiatorsystem.

2. Dentalmasse nach Anspruch 1, wobei a für 3 steht.

3. Dentalmasse nach Anspruch 1, wobei a für 2 steht.

4. Dentalmasse nach einem der Ansprüche 1 bis 3, wobei A für ein Alkylen steht.

5. Dentalmasse nach Anspruch 4, wobei A für ein Alkylen mit 3-10 Kohlenstoffatomen steht.

6. Dentalmasse nach einem der Ansprüche 1 bis 5, wobei n für 0 steht.

7. Dentalmasse nach einem der Ansprüche 1 bis 6, ferner umfassend Reaktanten eines sekundären Harzsystems aus der Gruppe bestehend aus Epoxidverbindungen mit aromatischen Gruppen, aliphatischen Gruppen, cycloaliphatischen Gruppen und Kombinationen davon.

8. Dentalmasse nach Anspruch 7, wobei das sekundäre Harzsystem ferner siliciumhaltige Reaktanten umfaßt.

9. Dentalmasse nach einem der Ansprüche 1 bis 8, ferner umfassend ein Füllstoffsystem.

10. Dentalmasse nach einem der Ansprüche 1 bis 9, ferner umfassend ein Additiv aus der Gruppe bestehend aus einem Farbmittel, einem oberflächenaktiven Mittel, einem Geschmacksverbesserer, einem Medikament, einem Stabilisator, einem Viskositätsmodifikator, einem Verdünnungsmittel, einem Fließadditiv, einem Thixotropiermittel, einem antimikrobiellen Mittel, einem polymeren Verdickungsmittel und Kombinationen davon.

**Revendications**

1. Composition dentaire comprenant :

un monomère éther à fonction époxy de formule (I) :

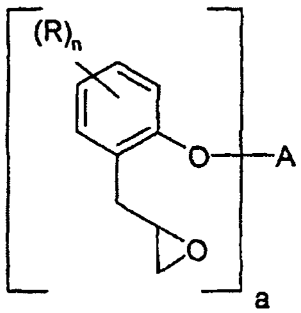

(I)

dans laquelle :

A représente un groupe alkylène à chaîne linéaire ou ramifiée, un groupe cycloalkylène, un groupe arylalkylène ou un groupe arylcycloalkylène, chacun renfermant de 1 à 30 atomes de carbone, où un ou plusieurs atomes de carbone et/ou d'hydrogène sont éventuellement substitués par un ou plusieurs atomes de brome, de chlore, d'azote ou d'oxygène, ou des combinaisons de ceux-ci ;
chaque R représente indépendamment un groupe alkyle, un groupe aryle ou un groupe époxyalkyle ;
n vaut de 0 à 4 ; et
a vaut de 2 à 4 ; et

un système amorceur.

2. Composition dentaire selon la revendication 1, dans laquelle a vaut 3.

3. Composition dentaire selon la revendication 1, dans laquelle a vaut 2.

4. Composition dentaire selon l'une quelconque des revendications 1 à 3, dans laquelle A représente un groupe alkylène.

5. Composition dentaire selon la revendication 4, dans laquelle A représente un groupe alkylène renfermant de 3 à 10 atomes de carbone.

6. Composition dentaire selon l'une quelconque des revendications 1 à 5, dans laquelle n vaut 0.

7. Composition dentaire selon l'une quelconque des revendications 1 à 6, comprenant en outre des réactifs à système de résine secondaire, choisis parmi le groupe constitué de composés époxy renfermant des groupes aromatiques, des groupes aliphatiques, des groupes cycloaliphatiques et leurs combinaisons.

8. Composition dentaire selon la revendication 7, dans laquelle le système de résine secondaire comprend en outre des réactifs siliciés.

9. Composition dentaire selon l'une quelconque des revendications 1 à 8, comprenant en outre un système de charge.

10. Composition dentaire selon l'une quelconque des revendications 1 à 9, comprenant en outre un additif choisi parmi le groupe constitué d'un colorant, d'un agent tensioactif, d'un agent aromatisant, d'un médicament, d'un agent stabilisant, d'un modificateur de la viscosité, d'un agent diluant, d'un additif de régulation de la fluidité, d'un agent thixotrope, d'un agent anti-microbien, d'un agent épaississant polymère et de leurs combinaisons.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9903444 A
- US 6187836 B, Oxman
- US 6084004 B, Weinmann
- US 6245828 B, Weinmann
- US 5037861 B, Crivello
- US 2003035899 A, Klettke
- US 3018262 A, Schroeder
- WO 0151540 A, Klettke
- US 20050113477 A1
- EP 0897710 A, Weinmann
- US 5856373 A, Kaisaki
- US 6084004 A, Weinmann
- US 6187833 A, Oxman
- US 6187836 A, Oxman
- US 20030166737 A, Dede
- US 4695251 A, Randklev
- US 4503169 A, Randklev
- US 6465641 B, Bretscher
- US 6387981 B, Zhang
- US 6572693 B, Wu
- WO 0130305 A, Zhang
- WO 0130306 A, Windisch
- WO 0130307 A, Zhang
- WO 03063804 A, Wu
- US 10847782 B
- US 10847781 B
- US 10847803 B
- US 6245528 B, Weinmann
- WO 2002066535 A, Klettke

### Non-patent literature cited in the description

- **LEE ; NEVILLE.** Handbook of Epoxy Resins. McGraw-Hill Book Co, 1967